# EUROPEAN PATENT APPLICATION

(11) **EP 4 498 379 A1**
(43) Date of publication of application: **29.01.2025**
(21) Application number: 23188144.2
(22) Date of filing: 27.07.2023
(51) Int. Cl.: G16H 30/40, G16H 50/70

(54) **MEDICAL IMAGE ANALYSIS**

(71) Applicant: Koninklijke Philips N.V., 5656 AG Eindhoven (NL)
(72) Inventor: NIE, Hongchao, Eindhoven (NL)
(74) Representative: Philips Intellectual Property & Standards

(57) **Abstract**

Proposed concepts aim to provide schemes, solutions, concepts, designs, methods, and systems pertaining to medical image analysis. In particular a difference between annotations relating to an anatomical object in first and second medical images of an area of interest is detected and used to generate a further annotation describing a difference between the anatomical objects. Such an annotation, based on the difference of the anatomical object in the medical images taken at different point in time, may be used for the training of a machine learning model adapted to identify differences in anatomical objects, or simply be provided to medical professionals and/or subjects.

## Description

### FIELD OF THE INVENTION

The present invention relates to the field of medical image analysis, and more particularly to analysing medical images of an area of interest of a subject and annotations describing an anatomical object in the area of interest.

### BACKGROUND OF THE INVENTION

As part of a typical workflow for a healthcare professional (e.g., a nurse, doctor, etc.), medical images are reviewed to create reports. For example, ultrasound images may be reviewed to generate annotated echocardiography reports. During reporting, users may indicate observations of the images in annotations organized by anatomical areas using structured finding codes. In many cases, users carry over information from prior reports of the subject (if there are any), and make changes when viewing new medical images. Nevertheless, the process of annotating the images is time consuming.

In general, noting the continuously increasing workload healthcare professionals are facing, it is desirable to automate any routine work of healthcare professionals, including the medical image annotation process. Many artificial intelligence-based solutions are currently being developed to aid in this objective. In the field of medical imaging, artificial intelligence models can pre-process images and detect abnormalities for clinicians to review the result for support of decision and/or diagnostic making.

However, one challenge to training said artificial intelligence models is to curate good quality training data. For instance, training data including annotated images which indicate what is observed on the images is highly desirable. Asking healthcare professionals to provide such data (in addition to performing their already busy job) is laborious and expensive. Thus, means for generation of more annotated data with reduced cost is needed.

### SUMMARY OF THE INVENTION

The invention is defined by the claims.

According to examples in accordance with an aspect of the invention, there is provided a computer-implemented method of medical image analysis. The method comprises:
obtaining a first medical image of an area of interest of a subject and a first annotation describing an anatomical object in the area of interest in the first medical image, the first medical image acquired at a first point in time;
obtaining a second medical image of the area of interest of the subject and a second annotation describing the anatomical object in the area of interest of the second medical image, the second medical image acquired at a second point in time subsequent to the first point in time;
detecting a difference between the first annotation and the second annotation;
generating an annotation describing a change in the anatomical object based on the detected difference; and
outputting the generated annotation.

By way of explanation, if a previous/historic medical image of an anatomical image exists, usually users will, when possible, consult the previous medical image and associated annotations when producing annotation(s) for a new/present medical image. That is, the user will usually edit annotations describing the anatomical object in the first medical image to describe the anatomical object in the second medical image. It has been realised that this process can be leveraged to generate a new annotation describing a change in the anatomical object. A difference between an annotation describing the anatomical object in the first image and an annotation describing the anatomical object in the second image can be used to generate an annotation describing a change in the anatomical object.

Such an annotation may be useful for users (e.g., medical professionals, subjects) to understand a change pertaining to an anatomical object over time, by means of a change in the anatomical object, quickly and easily. Further, the annotation may be useful for training machine learning algorithms configured to detect differences in anatomical objects between medical images.

For example, the first medical image may contain the heart of a subject as the anatomical object, and be associated with an annotation stating that the left ventricle is large. The second medical image may be received by a user, who begins the annotation process by obtaining the first medical image and associated annotation. The user then edits the annotation of the first medical image to generate an annotation corresponding to the second medical image, which may be for example that the left ventricle is of a normal size. The present invention will detect this difference between the first and the second annotation (e.g., "large" to "normal"), and generates an annotation based on the difference (e.g., "reduction in left ventricle size"). Accordingly, a useful annotation is provided that cites a difference between the medical images, which may be used for training a machine learning model, or for aiding an observer of the medical image(s) to understand a context of the image(s).

In some embodiments, the method may further comprise identifying a part of the first medical image corresponding to the anatomical object; generating a first partial image by segmenting the first medical image based on the identified part; identifying a part of the second medical image corresponding to the anatomical object; generating a second partial image by segmenting the second medical image based on the identified part; and outputting the first partial image and the second partial image

That is, known image recognition and segmentation techniques may be used to extract first and second partial images of the anatomical object of interest. Thus, the invention may provide first and second partial images alongside the generated annotation that provide a focussed view of the change indicated by the difference in annotations. Such a focussed image and associated annotation may be useful for providing a particularly understandable view of the first and second images for an observer, and may also be more useful for efficient training of a machine learning model configured to detect and annotate differences between medical images.

Also, the method may further comprise embedding the first partial image and/or the second partial image with the generated annotation.

Thus, the embedded image(s) and generated annotation can be provided to an observer and/or a training algorithm for a machine learning model.

Furthermore, identifying the part of the first medical image corresponding to the anatomical object may comprises determining the anatomical object by analysing the first annotation and/or second annotation; and locating the anatomical object within the area of interest of the first medical image by processing the first medical image using an image analysis model.

It may be beneficial for embodiments of the invention to automatically detect the anatomical object of interest by using the information of the first and/or second annotation. By improving automation, an ease of use of the invention is increased. That is, the first medical image and second medical image and corresponding annotations will simply need to be provided, without the need for a user to specify the anatomical object of interest (as this information is provided by the annotation).

In addition, identifying the part of the second medical image corresponding to the anatomical object may comprise determining the anatomical object by analysing the first annotation and/or second annotation; and locating the anatomical object within the area of interest of the second medical image by processing the second medical image using an image analysis model.

In some embodiments, the first annotation and/or the second annotation may comprise structured finding codes indicating the anatomical object and describing a conclusion related to that anatomical object.

Structured finding codes are typically used during report generation to facilitate a user providing annotations for the medical image. The difference between the structured finding codes may be relatively straightforward to detect and determine.

Exemplary embodiments may further comprise steps of prompting a user to input the second annotation; receiving the second annotation from the user; determining a confidence level associated with the generated annotation, the confidence level based at least one of: a number and/or type of annotations entered by the user before inputting the second annotation, a length of time between prompting the user and receiving the second annotation; and a character of the second annotation; and outputting the confidence level.

Advantageously, information relating to the way in which the user entered the annotation may be leveraged to predict an associated confidence in the annotation (and therefore a probable discernability of the difference). For example, if the user when entering the second annotation takes a long time to provide the second annotation, this may indicate that the difference is difficult to determine and/or may not be there at all. Also, if the user switches between many different annotations before deciding on a final annotation to provide, this may also indicate a low discernability level and/or a high likelihood that the difference isn't present. A confidence level based on the above may be useful to provide to future observer's contentious areas, and/or may be useful for training machine learning algorithms.

According to examples in accordance with a further aspect of the invention there is provided a computer-implemented method for training a medical image analysis model adapted to analyse medical images. The method comprises generating an annotation based on any embodiment described above; and training the medical image analysis model using a training algorithm configured to receive an array of training inputs and known outputs, wherein the training inputs comprise the first medical image and the second medical image, and the known output comprises the generated annotation.

The annotation generated according to the above embodiments may be particularly useful for training a medical image analysis model adapted to analyse medical images. Indeed, training data is sparse, and expensive to produce. It is desirable to avoid using medical professionals to produce more data in addition to performing their already busy and high stress job. Therefore, generating annotations according to the invention, and using such annotations to train a medical image analysis model may result in a more accurate medical image analysis model with lower cost.

According to further examples in accordance with an aspect of the invention there is provided a computer-implemented method for training a medical image analysis model adapted to analyse medical images, comprising: obtaining a first medical image of an area of interest of a subject and a first annotation describing an anatomical object in the area of interest in the first medical image, the first medical image acquired at a first point in time; obtaining a second medical image of the area of interest of the subject and a second annotation describing the anatomical object in the area of interest of the second medical image, the second medical image acquired at a second point in time subsequent to the first point in time; detecting a difference between the first annotation and the second annotation; generating an annotation describing a change in the anatomical object based on the detected difference; and training the medical image analysis using a training algorithm configured to receive an array of training inputs and known outputs, wherein the training inputs comprise the first medical image and the second medical image, and the known output comprises the generated annotation.

According to yet another aspect of the invention, there is provided a computer-implemented method of annotating a medical image, the method comprising: training a medical image analysis model according to an above embodiment;
obtaining a first medical image of an area of interest of a subject acquired at a first point in time; obtaining a second medical image of the area of interest of the subject acquired at a second point in time subsequent to the first point in time; and acquiring an annotation describing a change in an anatomical object within the area of interest based on inputting the first medical image and the second medical image to the generated medical image analysis model.

Thus, the above generated annotation can be used to generate/train a medical image analysis model, which may subsequently be used to automate the process of medical image annotation. Accordingly, time and cost associated with the annotation process may be saved.

Moreover, the invention also provides a computer program comprising computer program code means adapted, when said computer program is run on a computer, to implement any of the above-described methods.

According to examples in accordance with additional aspects of the invention there is provided a system for medical image analysis, comprising:
an interface configured to: obtain a first medical image of an area of interest of a subject and a first annotation describing an anatomical object in the area of interest in the first medical image, the first medical image acquired at a first point in time; obtain a second medical image of the area of interest of the subject and a second annotation describing the anatomical object in the area of interest of the second medical image, the second medical image acquired at a second point in time subsequent to the first point in time;
a processor configured to: detect a difference between the first annotation and the second annotation; generate an annotation describing a change in the anatomical object based on the detected difference; and output the generated annotation.

In some embodiments, the processor may comprise an image processing unit configured to: identify a part of the first medical image corresponding to the anatomical object; generate a first partial image by segmenting the first medical image based on the identified part; identify a part of the second medical image corresponding to the anatomical object; generate a second partial image by segmenting the second medical image based on the identified part; and output the first partial image and the second partial image.

Further, the processor may be further configured to embed the first partial image and/or the second partial image with the generated annotation.

Also, the interface may be further configured to prompt a user to input the second annotation; and receive the second annotation from the user. In this case, the processor may be further configured to: determine a confidence level associated with the generated annotation, the confidence level based at least one of: a number and/or type of annotations entered by the user before inputting the second annotation, a length of time between prompting the user and receiving the second annotation; and a character of the second annotation; and output the confidence level.

According to examples in accordance with a further aspect of the invention there is provided a method for operating a personal care device comprising a movable component and an actuator. The method comprises moving, by the actuator, the moveable component; and changing, by the movement, a polarization parameter of a polarization element, the polarization element configured to polarize light incident on the polarization element.

According to additional examples in accordance with another aspect of the invention there is provided a computer program product comprising computer program code means which, when executed on a computing device having a processing system, cause the processing system to perform all of the steps of the method for operating a personal care device comprising a movable component and an actuator according to an embodiment of the invention.

These and other aspects of the invention will be apparent from and elucidated with reference to the embodiments described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 presents a flow diagram of a method of medical image analysis according to an embodiment of the invention;
Fig. 2 presents a flow diagram of a method for training a medical image analysis model adapted to analyse medical images according to an aspect of another embodiment of the invention;
Fig. 3 presents a flow diagram of a method for annotating a medical image according to another aspect of the invention;
Fig. 4 is a simplified block diagram of a system for medical image analysis according to a further embodiment of the invention; and
Fig. 5 is a simplified block diagram of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to advantage.

It should be understood that the Figures are merely schematic and are not drawn to scale. It should also be understood that the same reference numerals are used throughout the Figures to indicate the same or similar parts.

Proposed concepts aim to provide schemes, solutions, concepts, designs, methods, and systems pertaining to medical image analysis. In particular a difference between annotations relating to an anatomical object in first and second medical images of an area of interest is detected and used to generate a further annotation describing a difference between the anatomical objects. Such an annotation, based on the difference of the anatomical object in the medical images taken at different point in time, may be used for the training of a machine learning model adapted to identify differences in anatomical objects, or simply be provided to medical professionals and/or subjects.

By way of explanation, various systems allow users (e.g., caregivers, healthcare professionals) to review medical images and create reports. During reporting, users use structured finding codes to indicate observations relating to anatomical objects present in the medical images of the subject. It has recently been observed that users typically carry over prior medical images and annotations of the subject (if there are any) and make changes when viewing new medical images. In other words, users add, remove, or modify the existing annotations (potentially in the form of finding codes) based on observations of the new medical image.

This process is time consuming, and subject to mistake which could lead to a wrong assessment of the evolution of an area of interest over time. Nevertheless, this process provides a good opportunity to follow the user's viewing process. Specifically, this provides an opportunity to track the real-time changes to reports that are carried over, and thus generate image delta annotations.

Accordingly, it is possible to generate a series of medical images related to an anatomical subject, labelled with corresponding annotations that show changes over time. This produces a technical benefit in which sequential changes in images are annotated behind the scenes of a normal reporting workflow. This may produce large volumes of ready-to-use training data for machine learning models for identifying changes in anatomical objects.

Put another way, the invention proposes a computer-implemented method (that could be artificial intelligence-based) where changes in annotated information (such as quantitative or qualitative measurement) corresponding to a subsequent medical image is automatically compared with the corresponding information (e.g., the image and associated annotation) of a prior medical image. This allows a user to quickly assess the evolution of a given situation over time. For example, this may enable the user to immediately ascertain a change in the anatomical object.

By way of example, an implementation of an embodiment of the invention may comprise the following workflow:
(i) The user "copies" a prior report (including a medical image of an area of interest of the subject, and an associated annotation of an anatomical object in the area of interest) as carry-over basis for a new report;
(ii) The prior medical image of the "carry-over" report is obtained for comparison with a new medical image;
(iii) The user reviews the new medical image and the prior medical image, and reports a change in an anatomical object by providing an annotation. For example, the user may change the finding code of a lower ventricle thrombus size from "large" to "small" (a simplified example, in real-life more technical terms may be used);
(iv) A change made to measurements/findings is detected compared to the prior report. Taking the last example, it is noted that the annotation has changed from "large" to "small";
(v) The anatomical part which the change describes (e.g., the left ventricle) is identified. This may be performed by looking up the term in an existing ontology;
(vi) An area of the prior medical image and new medical image corresponding to the anatomical part is located. This may be achieved by use of existing machine learning models for identification of anatomical objects. For instance, state-of-the-art models can detect whether a specified anatomical object are present in the image, and if yes, the location.
(vii) The located area is segmented out from both images. This results in focused images containing the anatomical part and the change of interest.
(viii) An annotation is generated from the annotations associated with the prior medical image and current medical image. That is, the annotations (e.g. "large" and "small") are translated to produce a change annotation (e.g., "thrombus size reduction").
(ix) The two segmented images are combined with the generated annotation, and stored for later use.

In addition to the above, embodiments of the invention may also provide that the order of changes is tracked. That is, an order in which the user changes annotations of the prior medical image to the annotations of the new medical image are noted. For example, the user may first change an annotation related to a left ventricle size, followed by changing the annotation related to a right ventricle side, and the invention tracks the order in which the user makes these changes. This may reflect the prominence of the reported findings (i.e. how obvious the change is to the eye of the trained user). The resulting indication of prominence may be used to train models that determine which change(s) in the anatomical object is more evident in the medical images, and which change(s) are less evident.

Furthermore, it may also be useful to track and record back-and-forth changes to annotations. A back-and-forth change may indicate uncertainty in the assessment of the user. For example, if it is detected that a change is modified back and forth (e.g., thrombus size from "large" to "medium" to "small" and then back to "medium"), then it may be useful to associate this behaviour to a confidence level. For instance, the change may be labelled as "size reduction from large to medium" with a confidence score 0.7. One method to calculate the score is to penalize by the number of changes made by the user. That is, for each edit, the confidence may be reduced by 0.1. In the example above, three edits lead to a score: 1 - 0.3 = 0.7. This may provide additional information about the user's confidence of the generated image annotation.

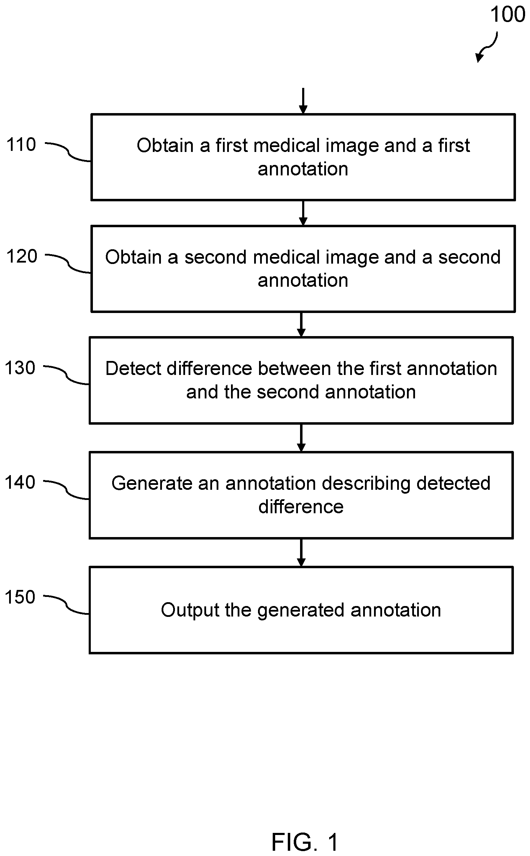

Moving on, Fig. 1 presents a flow diagram of a method of medical image analysis that is computer-implemented. That is, the method presented provides way of analysing one or more medical images (e.g., CT, MR, ultrasound images, etc.) of a subject (i.e., a patient). The method is computer-implemented, and therefore may be fully automated.

In step 110, a first medical image of an area of interest of a subject is obtained. The area of interest may be any part of the physical body of the subject, for example a chest area or a head area. The area of interest may be a 2D slice of the body of the subject, or may be a 3D reconstruction of the body of the subject. Within the area of interest is an anatomical object, such as an organ or part thereof. The first medical image is acquired (i.e., generated/captured) at a first point in time.

Also in step 110, a first annotation describing said anatomical object in the area of interest in the first medical image is obtained. The annotation may be any indication of a state, characteristic and/or property of the anatomical object that may be visible or indicated by a visible state of the anatomical object in the medical image (e.g., size, shape, condition, position, health, functionality, related pathologies, etc.).

Similarly, in step 120, a second medical image of the area of interest of the subject is obtained. Also in step 120, a second annotation describing the anatomical object in the area of interest of the second medical image is obtained.

However, the second medical image is acquired at a second point in time subsequent to the first point in time. That is the first medical image is an image of the anatomical object at a first point in time, and the second image is an image of the anatomical object at a second point in time after the first point in time.

The first medical image may be thought of as a historic/previous medical image, and the second medical image as a current/present medical image. However, embodiments are not restricted as such, and both the first medical image and second medical image may have been taken in the past (but at different times).

The first annotation and/or the second annotation may comprise or be in the form of structured finding codes indicating the anatomical object and describing a conclusion related to that anatomical object. Said structured finding codes are typically used to highlight areas of an image and a conclusion related to that image (e.g., organ X is enlarged).

In step 130, a difference between the first annotation and the second annotation is detected. That is, a change in the annotation describing the anatomical object in the first medical image, and the annotation describing the anatomical object in the second medical image is detected. This may be achieved by simply comparison, for example by using natural language processing.

In step 140, an annotation describing a change in the anatomical object is generated based on the detected difference. Thus, rather than having two annotations each describing a state, characteristic and/or property of the anatomical object in two different medical images, an annotation describing a change in the state, characteristic and/or property of the anatomical object between the first point in time and the second point in time is generated. For example, the first annotation may state that a pathology is present, the second annotation may state that the pathology is not present, and therefore the generated annotation may state that there has been a reduction in the presence of the pathology.

Finally, in step 150, the generated annotation is output. The generated annotation may be stored, or may be immediately output for viewing by a user. Additionally, or alternatively, the generated annotation may then be provided to a training algorithm for a medical image analysis model configured to determine changes in an anatomical object between images.

Of course, there may be more than one annotation relating to the anatomical object associated with each medical image. The above process may be performed for each annotation and a plurality of annotations generated.

Furthermore, the method may further comprise steps to generate first and second partial medical images. The partial medical images correspond to the part of the medical image in which the anatomical object is present. Therefore, an image focussed on the anatomical object of interest (and to which the annotation relates) is generated.

Firstly, a part of the first (and second) medical image corresponding to the anatomical object is identified. Then, a first partial image is generated by segmenting (i.e., cropping) the first medical image based on the identified part. Similarly, a second partial image is generated by segmenting the second medical image based on the identified part.

Identifying the part of the medical image may include determining the anatomical object by analysing the first annotation and/or second annotation (e.g., using a natural language processing algorithm). That is, the anatomical object is deduced from the annotation. The anatomical object is then located within the area of interest by processing the medical image using an image analysis model. Such models are well known and would be readily appreciated by the skilled person.

The first partial image and the second partial image may then be provided as output. That is, similarly to the annotation, the first and second partial image may be provided to an observer, be stored, or be provided to a training algorithm for a medical image analysis model.

In this vein, the first partial image and/or the second partial image may be embedded with the generated annotation. The generated annotation may be embedded or added to the partial image(s) as metadata, or may be visible printed onto the partial image(s).

Moreover, the method may also comprise determining a confidence level associated with the generated annotation. That is, the method may also generate an indication of a likelihood that the generated annotation accurately reflects the ground truth presented in the medical images. This is achieved by analysing the manner and/or context in which the user enters the second annotation.

Initially, the user is prompted to input the second annotation. The second annotation is then received from the user. During the time between the prompt and the annotation being received, a few pieces of data may be generated that can indicate a confidence in the second annotation. For example, a confidence level associated with the annotation may be determined based on a number and/or type of annotations entered by the user before inputting the second annotation (e.g., the user enters a number of different conclusions relating to the anatomical object before finalising the second annotation), a length of time between prompting the user and receiving the second annotation (e.g., indicative of a hesitation as the user attempts to deduce a conclusion related to the anatomical object), and a character of the second annotation (e.g., a tone and/or content of the second annotation may indicate how sure the user is about their finding). The confidence level may then be output similar to the generated annotation.

That is, a user may choose to start with the first annotation and revise it into the second annotation (i.e. edit a carried-over report). In this case, the second annotation may also comprise metadata describing a link with the first annotation. The result is a pair of annotations about the same anatomical part with different observation/conclusion values. This may be used to generate the annotation describing the difference in the anatomical part.

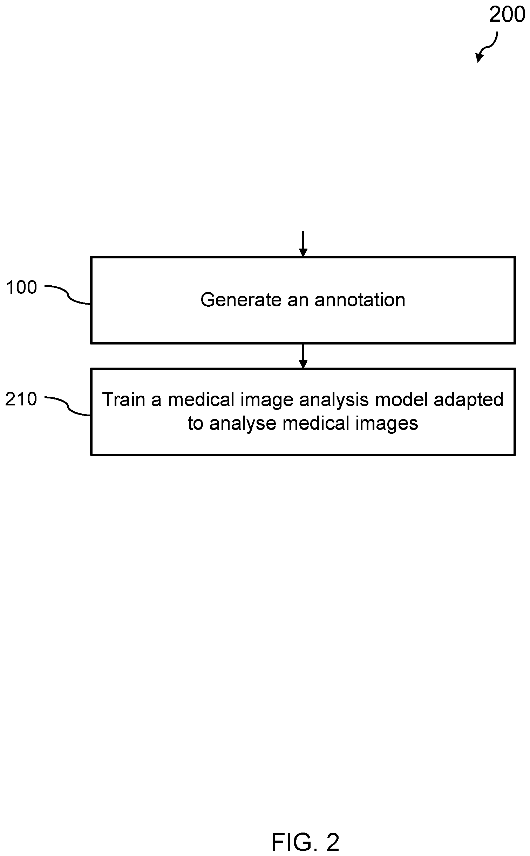

Fig. 2 presents a flow diagram of a method for training a medical image analysis model adapted to analyse medical images, which may be computer-implemented.

In step 100, an annotation is generated according to the method described in relation to Fig. 1. Repeated description of step 100 is therefore omitted for sake of brevity.

In step 210, the medical image analysis is trained using a training algorithm configured to receive an array of training inputs and known outputs. The training inputs comprise the first medical image and the second medical image, and the known output comprises the generated annotation.

Accordingly, the medical image analysis model may be trained to generate an annotation describing a difference in the anatomical object between the first and second medical image. Of course, this process may be performed for a plurality/array of different medical images and associated annotations to gradually improve the accuracy of the model. Some of the annotations may be created by a user (e.g., a medical professional), but at least one is generated according to the invention described in relation to Fig. 1.

Thus, the medical image analysis model may be useful in a number of different circumstances. For example, when the first annotation is carried over (i.e., used when a user is creating the second annotation), the medical image analysis model may automatically check the corresponding anatomical objects in the second medical image by invoking the image analysis model. If the model indicates an observation/conclusion related to the anatomical object different from the value of the first annotation, the user may be prompted to change the value corresponding to the first annotation to generate a second annotation. For example, in the user interface the field corresponding to the anatomical object and/or the first annotation may be highlighted to attract the user's attention.

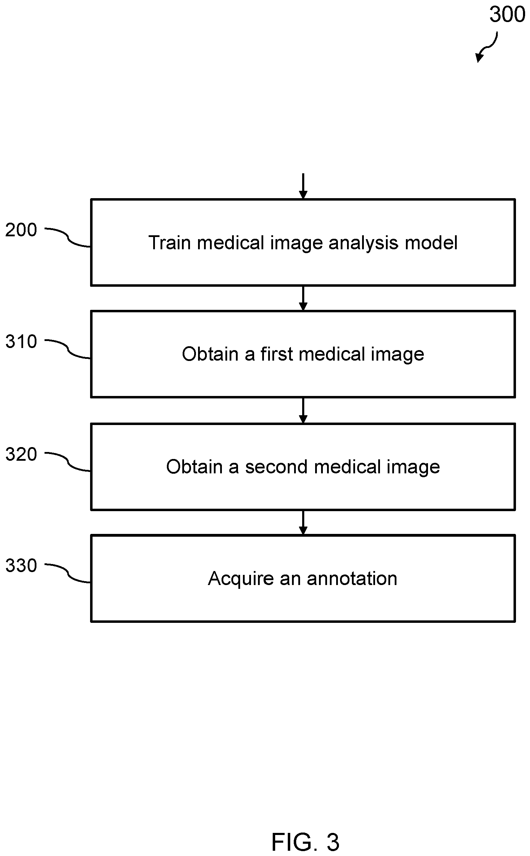

Fig. 3 presents a flow diagram of a method for annotating a medical image. The method begins with training a medical image analysis model adapted to analyse medical images according to a method described in relation to Fig. 2. Repeated description of step 200 is therefore omitted for sake of brevity. In other embodiments, the step 200 may be replaced by a step of generating the medical image analysis model including a step of training the medical image analysis model with the generated annotation.

In step 310, a first medical image of an area of interest of a subject acquired at a first point in time is obtained. In step 320, a second medical image of the area of interest of the subject acquired at a second point in time subsequent to the first point in time is obtained.

Then, in step 330, an annotation describing a change in an anatomical object within the area of interest is acquired. The annotation is acquired based on inputting the first medical image and the second medical image to the generated/trained medical image analysis model.

Accordingly, the medical image analysis model, trained based on generated annotations, may be used to automatically annotate sets of medical images of an anatomical object. This may provide a robust and accurate annotation, whilst saving the time of healthcare professionals.

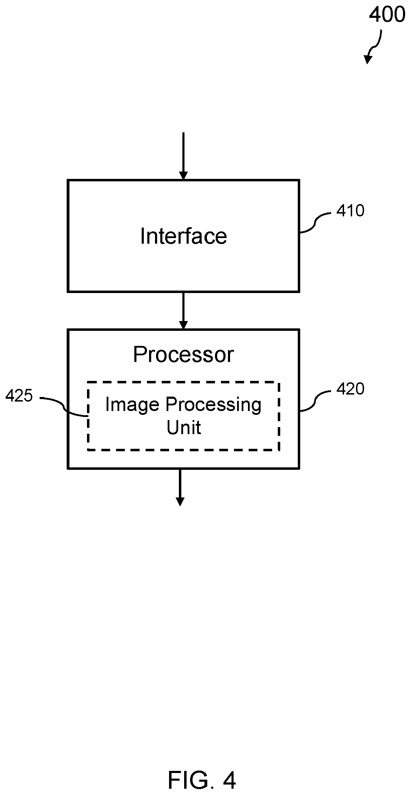

Fig. 4 provides a simplified block diagram of a system for medical image analysis. The system comprises an interface 410 and a processor 420. The processor may optionally comprise an image processing unit 425. Of course, the interface 410 and processor 420 may be implemented in the same device, or may be provided by separate devices.

The interface 410 is configured to obtain a first medical image of an area of interest of a subject and a first annotation describing an anatomical object in the area of interest in the first medical image. The interface 410 is also configured to obtain a second medical image of the area of interest of the subject and a second annotation describing the anatomical object in the area of interest of the second medical image.

Part of the interface 410 may be implemented as an image query module adapted to find the first medical image (i.e., prior medical image) reported by a carry-over report during annotation of the second medical image (i.e., new medical image).

The processor 420 is configured to detect a difference between the first annotation and the second annotation. The processor 420 then generates an annotation describing a change in the anatomical object based on the detected difference. Accordingly, the generated annotation is provided as output by the processor 420.

In some embodiments, the processor 420 comprises an image processing unit 425 configured to identify parts of the first medical image and second medical image corresponding to or including the anatomical object. The image processing unit 425 then generates first and second partial images by segmenting the first and second medical images, respectively, based on the identified parts.

Thus, the first and second partial images may be provided as output in addition to the annotation. Indeed, in further embodiments, the first partial image and/or the second partial image may be embedded with the generated annotation.

Furthermore, the interface 410 may be further configured to prompt a user to input the second annotation, and receive the second annotation from the user. This may be, for example, implemented in existing reporting software/systems.

The processor 420 may be further configured to determine a confidence level associated with the generated annotation. The confidence level (i.e., confidence value) may be based at least one of: a number and/or type of annotations entered by the user before inputting the second annotation, a length of time between prompting the user and receiving the second annotation; and a character of the second annotation.

Thus, the confidence level may be output by the processor 420 similarly to the annotation.

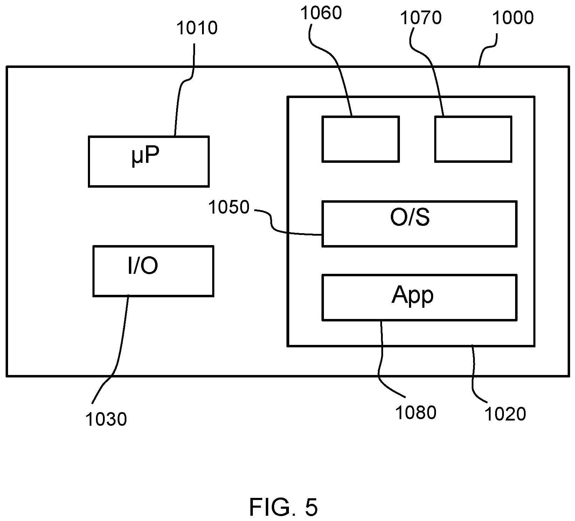

Fig. 5 illustrates an example of a computer 1000 within which one or more parts of an embodiment may be employed. Various operations discussed above may utilize the capabilities of the computer 1000. For example, one or more parts of a system for acquiring an input from a user to control an interface may be incorporated in any element, module, application, and/or component discussed herein. In this regard, it is to be understood that system functional blocks can run on a single computer or may be distributed over several computers and locations (e.g. connected via internet).

The computer 1000 includes, but is not limited to, smart phones, PCs, workstations, laptops, PDAs, palm devices, servers, storages, and the like. Generally, in terms of hardware architecture, the computer 1000 may include one or more processors 1010, memory 1020 and one or more I/O devices 1030 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 1010 is a hardware device for executing software that can be stored in the memory 1020. The processor 1010 can be virtually any custom made or commercially available processor, a central processing unit (CPU), a digital signal processor (DSP), or an auxiliary processor among several processors associated with the computer 1000, and the processor 1010 may be a semiconductor-based microprocessor (in the form of a microchip) or a microprocessor.

The memory 1020 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 1020 may incorporate electronic, magnetic, optical, and/or other types of storage media. Various storage media may be fixed within a processor or controller, may be transportable or may be available on-demand (e.g., via the cloud), such that the one or more programs stored thereon can be loaded into a processor or controller. Note that the memory 1020 can have a distributed architecture, where various components are situated remote from one another, but can be accessed by the processor 1010.

The software in the memory 1020 may include one or more separate programs, each of which comprises an ordered listing of executable instructions for implementing logical functions. The software in the memory 1020 includes a suitable operating system (O/S) 1050, compiler 1060, source code 1070, and one or more applications 1080 in accordance with exemplary embodiments. As illustrated, the application 1080 comprises numerous functional components for implementing the features and operations of the exemplary embodiments. The application 1080 of the computer 1000 may represent various applications, computational units, logic, functional units, processes, operations, virtual entities, and/or modules in accordance with exemplary embodiments, but the application 1080 is not meant to be a limitation.

The operating system 1050 controls the execution of other computer programs, and provides scheduling, input-output control, file and data management, memory management, and communication control and related services. It is contemplated by the inventors that the application 1080 for implementing exemplary embodiments may be applicable on all commercially available operating systems.

Application 1080 may be a source program, executable program (object code), script, or any other entity comprising a set of instructions to be performed. When a source program, then the program is usually translated via a compiler (such as the compiler 1060), assembler, interpreter, or the like, which may or may not be included within the memory 1020, so as to operate properly in connection with the O/S 1050. Furthermore, the application 1080 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, functional programming and the like.

The I/O devices 1030 may include input devices such as, for example but not limited to, a mouse, keyboard, scanner, microphone, camera, etc. Furthermore, the I/O devices 1030 may also include output devices, for example but not limited to a printer, display, etc. Finally, the I/O devices 1030 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 1030 also include components for communicating over various networks, such as the Internet or intranet.

If the computer 1000 is a PC, workstation, intelligent device or the like, the software in the memory 1020 may further include a basic input output system (BIOS) (omitted for simplicity). The BIOS is a set of essential software routines that initialize and test hardware at start-up, start the O/S 1050, and support the transfer of data among the hardware devices. The BIOS is stored in some type of read-only-memory, such as ROM, PROM, EPROM, EEPROM or the like, so that the BIOS can be executed when the computer 800 is activated.

When the computer 1000 is in operation, the processor 1010 is configured to execute software stored within the memory 1020, to communicate data to and from the memory 1020, and to generally control operations of the computer 1000 pursuant to the software. The application 1080 and the O/S 1050 are read, in whole or in part, by the processor 1010, perhaps buffered within the processor 1010, and then executed.

When the application 1080 is implemented in software it should be noted that the application 1080 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 1080 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods described in relation to Figs. 1-3, and the system described in relation to Fig. 4, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagram in Fig. 4 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

## Claims

1. A computer-implemented method of medical image analysis, comprising:
obtaining (110) a first medical image of an area of interest of a subject and a first annotation describing an anatomical object in the area of interest in the first medical image, the first medical image acquired at a first point in time;
obtaining (120) a second medical image of the area of interest of the subject and a second annotation describing the anatomical object in the area of interest of the second medical image, the second medical image acquired at a second point in time subsequent to the first point in time;
detecting (130) a difference between the first annotation and the second annotation;
generating (140) an annotation describing a change in the anatomical object based on the detected difference; and
outputting (150) the generated annotation.

2. The method of claim 1, further comprising:
identifying a part of the first medical image corresponding to the anatomical object;
generating a first partial image by segmenting the first medical image based on the identified part;
identifying a part of the second medical image corresponding to the anatomical object;
generating a second partial image by segmenting the second medical image based on the identified part; and
outputting the first partial image and the second partial image

3. The method of claim 2, further comprising embedding the first partial image and/or the second partial image with the generated annotation.

4. The method of claim 2 or 3, wherein identifying the part of the first medical image corresponding to the anatomical object comprises:
determining the anatomical object by analysing the first annotation and/or second annotation; and
locating the anatomical object within the area of interest of the first medical image by processing the first medical image using an image analysis model.

5. The method of any of claims 2-4, wherein identifying the part of the second medical image corresponding to the anatomical object comprises:
determining the anatomical object by analysing the first annotation and/or second annotation; and
locating the anatomical object within the area of interest of the second medical image by processing the second medical image using an image analysis model.

6. The method of any of claims 1-5, wherein the first annotation and/or the second annotation comprise structured finding codes indicating the anatomical object and describing a conclusion related to that anatomical object.

7. The method of any of claims 1-6, further comprising:
prompting a user to input the second annotation;
receiving the second annotation from the user;
determining a confidence level associated with the generated annotation, the confidence level based at least one of: a number and/or type of annotations entered by the user before inputting the second annotation, a length of time between prompting the user and receiving the second annotation; and a character of the second annotation; and
outputting the confidence level.

8. A computer-implemented method for training a medical image analysis model adapted to analyse medical images, comprising:
generating (100) an annotation based on any of claims 1-7; and
training (210) the medical image analysis model using a training algorithm configured to receive an array of training inputs and known outputs, wherein the training inputs comprise the first medical image and the second medical image, and the known output comprises the generated annotation.

9. A computer-implemented method for training a medical image analysis model adapted to analyse medical images, comprising:
obtaining (110) a first medical image of an area of interest of a subject and a first annotation describing an anatomical object in the area of interest in the first medical image, the first medical image acquired at a first point in time;
obtaining (120) a second medical image of the area of interest of the subject and a second annotation describing the anatomical object in the area of interest of the second medical image, the second medical image acquired at a second point in time subsequent to the first point in time;
detecting (130) a difference between the first annotation and the second annotation;
generating (140) an annotation describing a change in the anatomical object based on the detected difference; and
training (210) the medical image analysis using a training algorithm configured to receive an array of training inputs and known outputs, wherein the training inputs comprise the first medical image and the second medical image, and the known output comprises the generated annotation.

10. A computer-implemented method of annotating a medical image, the method comprising:
training (200) a medical image analysis model according to claims 8 or 9;
obtaining (310) a first medical image of an area of interest of a subject acquired at a first point in time;
obtaining (320) a second medical image of the area of interest of the subject acquired at a second point in time subsequent to the first point in time;
acquiring (330) an annotation describing a change in an anatomical object within the area of interest based on inputting the first medical image and the second medical image to the trained medical image analysis model.

11. A computer program comprising computer program code means adapted, when said computer program is run on a computer, to implement the method of any of claims 1-10.

12. A system for medical image analysis, comprising:
an interface (410) configured to:
obtain a first medical image of an area of interest of a subject and a first annotation describing an anatomical object in the area of interest in the first medical image, the first medical image acquired at a first point in time;
obtain a second medical image of the area of interest of the subject and a second annotation describing the anatomical object in the area of interest of the second medical image, the second medical image acquired at a second point in time subsequent to the first point in time;
a processor (420) configured to:
detect a difference between the first annotation and the second annotation;
generate an annotation describing a change in the anatomical object based on the detected difference; and
output the generated annotation.

13. The system of claim 12, wherein the processor (420) comprises an image processing unit (425) configured to:
identify a part of the first medical image corresponding to the anatomical object;
generate a first partial image by segmenting the first medical image based on the identified part;
identify a part of the second medical image corresponding to the anatomical object;
generate a second partial image by segmenting the second medical image based on the identified part; and
output the first partial image and the second partial image.

14. The system of claim 13, wherein the processor (420) is further configured to embed the first partial image and/or the second partial image with the generated annotation.

15. The system of any of claims 12-14, wherein the interface (410) is further configured to:
prompt a user to input the second annotation;
receive the second annotation from the user, and
wherein the processor (420) is further configured to:
determine a confidence level associated with the generated annotation, the confidence level based at least one of: a number and/or type of annotations entered by the user before inputting the second annotation, a length of time between prompting the user and receiving the second annotation; and a character of the second annotation; and
output the confidence level.
